# EUROPEAN PATENT SPECIFICATION

(11) **EP 1 287 837 B1**
(45) Date of publication and mention of the grant of the patent: **10.05.2006**
(21) Application number: 02018013.9
(22) Date of filing: 12.08.2002
(51) Int. Cl.: A61L 15/28

(54) **Absorbent article with film-like region of chitosan material and process for making the same**
Atmungsaktive absorbierende Artikel mit filmähnlichem Chitosanmaterial und Verfahren zu deren Herstellung
Article absorbant comprenant une région de chitosane semblable à une couche et procédé de préparation

(30) Priority: 24.08.2001 EP 01120342
(43) Date of publication of application: 05.03.2003
(73) Proprietor: THE PROCTER & GAMBLE COMPANY, Cincinnati, Ohio 45202 (US)
(72) Inventor: Tamburro, Maurizio, 65100 Pescara (IT); D'Alesio, Nicola, 66010 Canosa Sannita (Chieti) (IT); Pesce, Antonella, 65125 Pescara (IT); Di Cinto, Achille, 65126 Pescara (IT); Carlucci, Giovanni, 66100 Chieti (IT); Tordone, Adelia Alessandra, 65125 Pescara (IT)
(74) Representative: Kremer, Véronique Marie Joséphine

(56) References cited:
- EP-A- 0 393 825
- WO-A-99/32697
- US-A- 4 373 519
- US-A- 5 599 916
- DATABASE WPI Section Ch, Week 199249 Derwent Publications Ltd., London, GB; Class A96, AN 1992-404377 XP002186793 & JP 04 303445 A (NIPPON ZEON KK), 27 October 1992 (1992-10-27)

## Description

### FIELD OF THE INVENTION

The present invention relates to an absorbent article with an absorbent member, said member having a film-like region comprising chitosan material, and a process for making said absorbent member.

### BACKGROUND OF THE INVENTION

Webs, particularly fibrous structures for absorbing fluids, are manufactured for many uses. They are for example incorporated into absorbent articles such as disposable diapers, incontinent pads, sanitary napkins and panty liners as fluid absorption and/or fluid transmission and/or diffusion elements, especially as absorbent cores that are intended to absorb and retain body fluids.

The primary focus of absorbent articles is the ability of those articles to absorb and retain fluids. Indeed there is a continuous trend to be noted towards further increasing the fluid retention and leakage prevention characteristics of absorbent articles. However, presently available absorbent articles are still not completely satisfactory in this respect, since the occurrence of fluid leakage, e.g. through the topsheet or along the peripheral edge, is still a problem.

Besides those primary characteristics, other features like comfort are increasingly important for users of absorbent articles. To satisfy this need so-called breathable absorbent articles, offering air and vapour exchange through the backsheet of the articles, have been developed and commercialised. However, breathable articles suffer from negatives like occurrence of undesired body fluid leakage especially through the backsheet.

Attempts have been made to overcome these recurring problems, by for example increasing the amount of superabsorbent materials or using coagulants, see for example EP-A-906 074. However, these solutions are not completely satisfactory. It has been observed that despite the extensive use of superabsorbents, such as absorbent gelling materials, in absorbent articles in many incidents there was free fluid, especially between the backsheet and the absorbent core in used absorbent articles, which led to leakage through the topsheet or along the peripheral edge of the article. In the case of so-called breathable absorbent articles, the presence of this free fluid might additionally cause leakage through the breathable backsheet. Thus, there still exists the need for further improved absorbent articles, in which the generation of such free fluids and the leakage resulting therefrom is reliably inhibited.

It is therefore an object of the present invention to provide an absorbent article comprising an absorbent member offering improved fluid retention by preventing or at least reducing the occurrence of free fluids and/or immobilizing such free fluids. More particularly, the present invention seeks to provide absorbent articles having an absorbent member with improved fluid retention characteristics, resulting in reduced leakage.

It is a further object of the present invention to provide an absorbent article with an absorbent member in a particularly cost efficient way by using only a very low amount of active material.

The above-mentioned objects have now surprisingly been met by providing an absorbent article with an absorbent member comprising in at least one film-like region comprising particles of chitosan material. Said particles of chitosan material have a particle size distribution with a mean diameter D(v,0.9) of not more than about 300 µm.

It has been found that by providing at least one film-like region of particles of chitosan material as claimed the formation of free fluid in that region can be prevented through gelification of said fluid by the chitosan material. In particular it has been found that by selecting very fine particles of chitosan material, having a particles size distribution with a mean diameter D(v,0.9) of not more than about 300 µm, it is possible to obtain a high active surface of the chitosan material while using a reduced amount of the chitosan material. It has furthermore been found that by the film-like application of the chitosan material a continuous and homogeneous coverage is achieved in said film-like region with a reduced amount of chitosan material. Thus, it is possible to obtain a homogenous and continuous fluid-immobilizing functionality in a predetermined region of an absorbent article while using less chitosan material compared to the amount of chitosan material, which is needed to obtain such a continuous and homogeneous coverage with a non-film-like application of chitosan material. The homogenous and continuous coverage of chitosan material in a certain region of an absorbent article ensures that free fluid in said region is immobilized and is prevented from leaking to the outside of the absorbent article.

In a preferred embodiment, the film-like region of particles of chitosan material also optimises the prevention of fluid leakage in breathable absorbent articles. In a particularly preferred embodiment, the absorbent member according to the present invention is made of substantially hydrophilic material for optimum liquid absorption, liquid acquisition and/or liquid handling. This is particularly important when the absorbent member according to the present invention is used as absorbent core or secondary topsheet or secondary backsheet in absorbent articles. It has been found that by immobilizing occurring free fluids these fluids are prevented from reaching the parts of the absorbent article between the absorbent core and the breathable backsheet. By this, the probability of leakage through the breathable backsheet is significantly reduced.

In its broadest embodiment, the present invention also encompasses a process of making an absorbent member, wherein a solution or dispersion of chitosan material is applied onto a precursor web in the form of a spray of droplets, said droplets having a droplet size distribution with a mean diameter D(v,0.9) of not more than 1500 µm. An advantage of this process is that a film-like region of particles of chitosan materials is provided on the surface of the precursor web, which translates in outstanding fluid retention / leakage prevention towards fluids while requiring a lower amount of chitosan material. Indeed, by applying a solution or dispersion of chitosan materials onto the precursor web in the form of a spray of small droplets as defined herein, a higher coverage can be achieved with the same amount of solution/dispersion as compared to applying the same solution or dispersion in the form of a spray of droplets with larger droplets. Furthermore, applying the solution or dispersion onto the precursor web as small droplets also translates into limited wetting of the surface and thus in better processability of the web and subsequent disposable absorbent articles comprising the resulting absorbent member.

### PRIOR ART BACKGROUND

The use of chitosan in absorbent articles has been discussed in several prior art documents. EP-B-627,225 discloses the preparation of chitosan compounds with improved absorption characteristics and suggests their usage in sanitary hygiene articles. DE 19,913,478 discloses a breast pad comprising chitosan for improved absorption of fat-containing liquids, such as milk. WO 99/61079 and WO 99/32697 disclose the use of chitosan coatings onto hydrophobic substrates for providing antimicrobial absorbent structures, e.g. nonwovens. EP-B-393,825 teaches the utilization of chitosan salts in absorbent products. A structure formed by a cellulose web containing chitosan for water absorbance and starch as the binder for the structure is disclosed.

None of the cited prior art discloses an absorbent article with an absorbent member comprising at least one film-like region of chitosan material, let alone the benefits associated thereby, namely leakage reduction and improved retention at reduced consumption of chitosan material.

### SUMMARY OF THE INVENTION

The present invention relates to an absorbent article with an absorbent member, said member having a film-like region of particles of chitosan material as claimed in claim 1. Said absorbent member is suggested to be used as absorbent core or secondary topsheet or secondary backsheet in absorbent articles of personal hygiene.

The present invention furthermore encompasses a process for making an absorbent member comprising at least one film-like region of particles of chitosan material. The method comprises the essential steps of forming a precursor web, applying a solution or dispersion of chitosan material onto the precursor web and forming a film-like region of particles of chitosan material on the precursor web upon drying the precursor web, whereby said solution or dispersion is applied as a spray of droplets having a droplet size distribution with a mean diameter D(v,0.9) of not more than about 1500 µm.

### BRIEF DESCRIPTION OF THE DRAWINGS

Figure 1 illustrates one possible configuration of the process for making the absorbent member according to the present invention.
Figure 2 shows an exemplary ESEM (Environmental Scanning electronic microscope) picture (30x magnification) of a surface of an absorbent member according to the present invention, having applied latex onto it and chitosan material on top of the latex. Film-like regions of particles of chitosan material can be recognized.
Figure 3 shows an exemplary ESEM picture (20,000x magnification) of an absorbent member according to the present invention, wherein the particle size of the chitosan material and the film-like character of the applied pattern of particles of chitosan material can be seen.

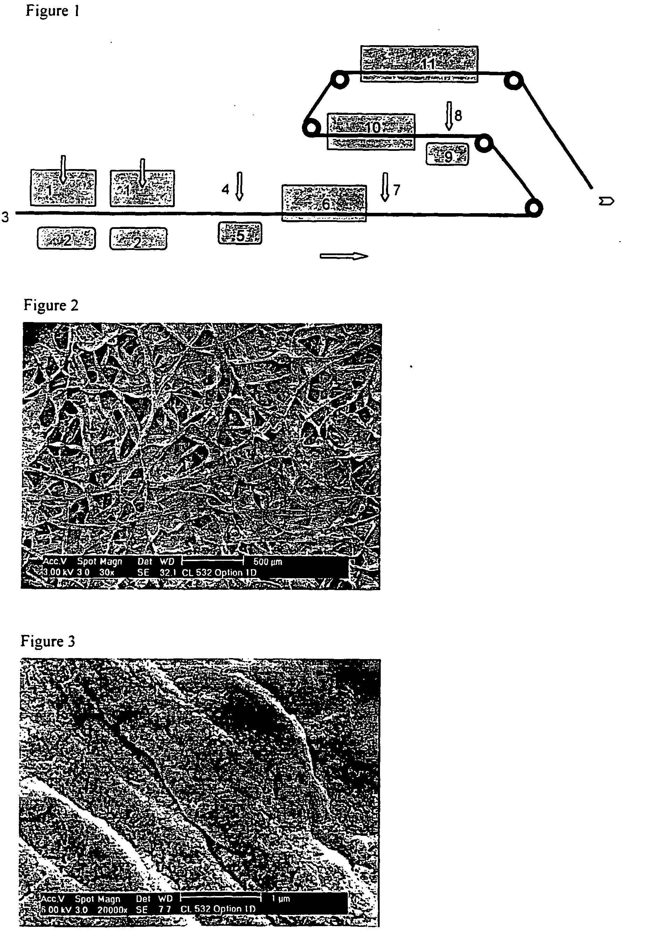

The Figures 2 and 3 were taken from a Philips XL30 ESEM FEG apparatus.

### DETAILED DESCRIPTION OF THE INVENTION

### Definitions

The term 'absorbent article' is used herein in a very broad sense including any article able to receive and/or absorb and/or contain and/or retain fluids and/or exudates, especially bodily fluids/bodily exudates. The absorbent article, which is referred to in the present invention typically comprises a fluid pervious topsheet, a fluid impervious backsheet that is preferably water vapour and/or gas pervious and an absorbent core comprised there between. Particularly preferred absorbent articles in the context of the present invention are disposable absorbent articles. Typical disposable absorbent articles according to the present invention are diapers, surgical and wound dressings and perspiration pads, incontinence pads, and preferably absorbent articles for feminine hygiene like sanitary napkins, panty liners, tampons, interlabial devices or the like.

The term 'disposable' is used herein to describe articles, which are not intended to be laundered or otherwise restored or reused as an article (i.e. they are intended to be discarded after a single use and preferably to be recycled, composted or otherwise disposed of in an environmentally compatible manner).

The term 'use', as used herein, refers to the period of time that starts when the absorbent article is actually put in contact with the anatomy of the user.

### Absorbent member for use in absorbent articles

The term 'absorbent member' is used herein to describe absorbent webs suitable for use in absorbent articles. The absorbent member comprises two surfaces aligned substantially opposite to each other. The first and the second surface are spaced apart from each other by the thickness dimension of the absorbent member. The absorbent member comprises at least one film-like region of particles of chitosan material and optionally a latex coating. The absorbent member according to the present invention can be used as absorbent core or so-called secondary topsheet or secondary backsheet in absorbent articles. The absorbent member typically has significant internal void space in the form of pores, holes, apertures, interstitial space between fibres and the like. Examples of absorbent members for use in the present invention are fibrous webs, such as nonwovens or fabrics, comprising natural or synthetic fibres or mixtures thereof, or apertured polymeric films or foam materials. Indeed, the absorbent member according to the present invention can be made of any of a variety of fibres, including a blend or admixture. The fibres may be cellulosic, modified cellulosic, or hydrophilic synthetic and include such fibres as wood pulp, rayon, cotton, cellulose acetate, polyester, nylon and the like. The absorbent member can be made according to any suitable method known for this purpose in the art. Fibrous absorbent members according to the present invention can be made by appropriate processes such as dry laying and in particular air laying, melt blowing or spunbonding. Film-like or foam-like absorbent members according to the present invention are made by processes suitable for such purposes. Highly preferred absorbent members for use herein are hydrophilic fibrous webs. As used herein, 'hydrophilic' refers to a material having a contact angle of water in air of less than 90 degrees, whereas the term 'hydrophobic' herein refers to a material having a contact angle of water in air of 90 degrees or greater. An absorbent member comprising hydrophilic fibres like for example cellulosic fibres such as wood pulp fibres is particularly useful in such products as sanitary napkins, disposable diapers or wipes because the hydrophilic fibres are liquid absorbent and therefore enhance the overall absorbency of the absorbent member. Preferably, absorbent members for use herein can be made of a blend of cellulosic and hydrophilic synthetic fibres, typically comprising about 65% to 95% by weight of cellulosic fibres and more preferably up to about 20% by weight of the hydrophilic synthetic fibres. The hydrophilic synthetic fibres, which can be provided in any length including staple length, can improve the strength of the absorbent member. Hydrophobic fibres or films, such as fibres or films made of polyethylene or polypropylene, may also be used in the absorbent member herein provided they are treated by e.g. surfactants to make them hydrophilic, in order not to decrease the absorbent capacity of the preferred absorbent member.

'Secondary topsheet' as used herein means layers in absorbent articles, which are located between the absorbent core and the topsheet of the article. Respectively, 'secondary backsheet' as used herein means layers in absorbent articles, which are located between the absorbent core and the backsheet of the article.

In a preferred embodiment, the absorbent member according to the present invention is used in the absorbent core of an absorbent article. The film-like region of particles of chitosan material has the property of gelifying free fluid, which often occurs between the absorbent core and the backsheet and thereby prevents the fluid from leaking to the outside of the absorbent article.

In another preferred embodiment, the absorbent member according to the present invention is used in breathable absorbent articles. In this embodiment it is preferred that the region of the absorbent member according to the present invention, which comprises chitosan material in a film-like pattern, is preferably directed away from the wearer's skin towards the garment of the wearer. In other words, the chitosan material-comprising film-like region is preferably directed towards the garment-facing surface of the breathable absorbent article. By this construction, a fluid barrier towards the backsheet of the absorbent article is established, which by gelification hinders or even prevents body fluids from approaching the breathable backsheet.

The absorbent member according to the present invention comprises as essential feature at least one film-like region of particles of chitosan material. By 'chitosan material', it is meant herein chitosans, modified chitosans, crosslinked chitosans, chitosan salts or mixtures thereof.

Chitosan is a partially or fully deacetylated form of chitin, a naturally occurring polysaccharide. Indeed, chitosan is an aminopolysaccharide usually prepared by deacetylation of chitin (polybeta(1,4)-N-acetyl-D-glucosamine).

Chitosan is not a single, definite chemical entity but varies in composition depending on the conditions of manufacture. It may be equally defined as chitin sufficiently deacetylated to form soluble amine salts. Chitosan is the beta-(1,4)-polysaccharide of D-glucosamine and is structurally similar to cellulose, except that the C-2 hydroxyl group in cellulose is substituted with a primary amine group in chitosan. The large number of free amine groups makes chitosan a polymeric weak base. Solutions of chitosan are generally highly viscous, resembling those of natural gums.

The chitosan used herein is suitably in relatively pure form. Methods for the manufacture of pure chitosan are well known. Generally, chitin is milled into a powder and demineralised with an organic acid such as acetic acid. Proteins and lipids are then removed by treatment with a base, such as sodium hydroxide, followed by chitin deacetylation by treatment with concentrated base, such as 40 percent sodium hydroxide. The chitosan formed is washed with water until the desired pH is reached.

The properties of chitosan relate to its polyelectrolyte and polymeric carbohydrate character. Thus, it is generally insoluble in water, in alkaline solutions at pH levels above about 7, or in hydrophobic organic solvents. It generally dissolves readily in dilute aqueous solutions of organic acids such as formic, acetic, tartaric, glycolic, lactic and citric acids and also in dilute aqueous solutions of mineral acids, except, for example, sulphuric acid. In general, the amount of acid required to dissolve chitosan is approximately stoichiometric with the amino groups. Since the pKₐ for the amino groups present in chitosan is between 6.0 and 7.0, they can be protonated in very dilute acids or even close to neutral conditions, rendering a cationic nature to this biopolymer. This cationic nature is the basis of many of the benefits of chitosan. Indeed, chitosan material can be considered as a linear polyelectrolyte with a high charge density which can interact with negatively charged surfaces, like proteins (e.g. by interfering with the proteinic wall construction of microorganisms, thereby acting as an antimicrobial agent and/or by reacting with the proteins present in bodily fluid, like menses, thereby acting as a gelifying agent for such fluid).

Without wishing to be bound by any theory, it is believed that chitosan material retains electrolyte-containing fluids like body fluids by multiple mechanisms.

One mechanism is conventional absorption by incorporation of the water dipole molecules into the structure. As the quaternary ammonium groups, being positively charged, are distracting each other, molecular cavities exist, in which water molecules can penetrate. By the penetration of dipole molecules, like water, these cavities can be widened by swelling and thereby generating even more space for further water molecules. This mechanism can be continued until the limits of molecular tension are reached.

The second mechanism of binding electrolyte-containing fluids, like body fluids, by chitosan material is gelification. Chitosan material acts electrostatically on nearby negatively charged molecules and thereby holds them in its circumference. The positively charged cationic groups (e.g., quaternary ammonium groups) of the chitosan material will interact with negatively charged anionic function-bearing molecules present in bodily fluids, like for example the carboxylic groups of proteins. This will result in the formation of a three-dimensional network between the chitosan material and such molecules with anionic groups (gelification of the bodily fluids). This gelification will further entrap other molecules present in body fluids (like lipids, acids). Due to the gelification properties of the chitosan material with respect to electrolyte-containing fluids, a liquid barrier is generated when the chitosan material is wetted by such fluids.

Preferred chitosan materials for use herein have an average degree of deacetylation (D.A.) of more than 70%, preferably from 80% to about 100%. The degree of deacetylation refers to the percentage of the amine groups that are deacetylated. This characteristic is directly related to the hydrogen bonding existing in this biopolymer, affecting its structure, solubility and ultimately its reactivity. The degree of deacetylation can be determined, by titration, dye adsorption, UV/vis, IR and NMR spectroscopy. The degree of deacetylation will influence the cationic properties of chitosan. By increasing the degree of deacetylation the cationic character of the chitosan material will increase and thus also its gelifying abilities.

Suitable chitosan materials to use herein include substantially water-soluble chitosan. As used herein, a material will be considered water-soluble when it substantially dissolves in excess water to form a clear and stable solution, thereby, losing its initially particulate form and becoming essentially molecularly dispersed throughout the water solution. Preferred chitosan materials for use herein are water soluble, i.e. at least 1 gram and preferably at least 3 gram of the chitosan materials are soluble in 100 grams of water at 25°C and one atmosphere. By 'solubility' of a given compound it is to be understood herein the amount of said compound solubilised in deionised water at 25°C and one atmosphere in absence of a precipitate. Generally, the water-soluble chitosan materials will be free from a higher degree of crosslinking, as crosslinking tends to render the chitosan materials water insoluble.

Chitosan materials may generally have a wide range of molecular weights. Chitosan materials with a wide range of molecular weights are suitable for use in the present invention. Typically, chitosan materials for use herein have a molecular weight ranging from 1,000 to 10,000,000 grams per gram moles and more preferably from 2,000 to 1,000,000. Molecular weight means average molecular weight. Methods for determining the average molecular weight of chitosan materials are known to those skilled in the art. Typical methods include for example light scattering, intrinsic viscosity and gel permeation chromatography. It is generally most convenient to express the molecular weight of a chitosan material in terms of its viscosity in a 1.0 weight percent aqueous solution at 25°C with a Brookfield viscometer. It is common to indirectly measure the viscosity of the chitosan material by measuring the viscosity of a corresponding chitosan salt, such as by using a 1.0 weight percent acetic acid aqueous solution. Chitosan materials suitable for use in the present invention will suitably have a viscosity in a 1.0 weight-% aqueous solution at 25°C of from about 10 mPa·s (10 centipoise) to about 100,000 mPa·s (100,000 centipoise), more suitably from about 30 mPa·s (30 centipoise) to about 10,000 mPa·s (10,000 centipoise), even more suitably 7000 mPa·s (7000 centipoise).

The pH of the chitosan materials depends on their preparation. Preferred chitosan materials for use herein have an acidic pH, typically in the range of 3 to 7, preferably about 5. By pH of the chitosan material, it is meant herein the pH of a 1% chitosan material solution (1 gram of chitosan material dissolved in 100 grams of distilled water) measured by a pH-meter. By using a more acidic pH, the cationic character of the chitosan materials will be increased and thus their gelifying abilities. However, too high acidity is detrimental to skin safety. Thus it is highly preferred herein to use chitosan materials with a pH of about 5, thereby delivering the best compromise between fluid handling properties on one side and skin compatibility on the other side.

Particularly suitable chitosan materials for use herein are chitosan salts, especially water-soluble chitosan salts. A variety of acids can be used for forming chitosan salts. Suitable acids for use are soluble in water or partially soluble in water, are sufficiently acidic to form the ammonium salt of chitosan and yet not sufficiently acidic to cause hydrolysis of chitosan and are present in amount sufficient to protonate the reactive sites of chitosan.

Preferred acids can be represented by the formula:

R-(COOH)ₙ

wherein n has a value of 1 to 3 and R represents a mono- or divalent organic radical composed of carbon, hydrogen and optionally at least one of oxygen, nitrogen and sulphur or simply R is an hydrogen atom. Preferred acids are the mono- and dicarboxylic acids composed of carbon, hydrogen,oxygen and nitrogen (also called hereinafter amino acids). Such acids are highly desired herein as they are biologically acceptable for use against or in proximity to the human body. Illustrative acids, in addition to those previously mentioned include, among others, are citric acid, formic acid, acetic acid, N-acetylglycine, acetylsalicylic acid, fumaric acid, glycolic acid, iminodiacetic acid, itaconic acid, lactic acid, maleic acid, malic acid, nicotinic acid, 2-pyrrolidone-5-carboylic acid, salycilic acid, succinamic acid, succinic acid, ascorbic acid, aspartic acid, glutamic acid, glutaric acid, malonic acid, pyruvic acid, sulfonyldiacetic acid, benzoic acid, epoxysuccinic acid, adipic acid, thiodiacetic acid and thioglycolic acid. Any chitosan salts formed from the reaction of chitosan with any of these acids are suitable for use herein.

Examples of chitosan salts formed with an inorganic acid include, but are not limited to, chitosan hydrochloride, chitosan hydrobromide, chitosan phosphate, chitosan sulphonate, chitosan chlorosulphonate, chitosan chloroacetate and mixtures thereof. Examples of chitosan salts formed with an organic acid include, but are not limited to, chitosan formate, chitosan acetate, chitosan lactate, chitosan glycolate, chitosan malonate, chitosan epoxysuccinate, chitosan benzoate, chitosan adipate, chitosan citrate, chitosan salicylate, chitosan propionate, chitosan nitrilotriacetate, chitosan itaconate, chitosan hydroxyacetate, chitosan butyrate, chitosan isobutyrate, chitosan acrylate and mixtures thereof. It is also suitable to form a chitosan salt using a mixture of acids including, for example, both inorganic and organic acids.

Highly preferred chitosan salts for use herein are those formed by the reaction of chitosan with an amino acid. Amino acids are molecules containing both an acidic and amino functional group. The use of amino acids is highly preferred as those chitosan amino salts have higher skin compatibility. Indeed most of the amino acids are naturally present on the skin. Chitosan salts of pyrrolidone carboxylic acid are effective moisturizing agents and are non-irritating to skin. Amino acids for use herein include both linear and/or cyclo amino acids. Examples of amino acids for use herein include, but are not limited to, alanine, valine, leucine, isoleucine, prolinephenylalanine, triptofane, metionine, glycine, serine, cysteine, tyrosine, asparagine, glutamine, aspartic acid, glutamic acid, lysine, arginine, istydine, hydroxyproline and the like. A particularly suitable example of a cyclic amino acid is pyrrolidone carboxylic acid, which is a carboxylic acid of pyrrolidin-2-one as per following formula:

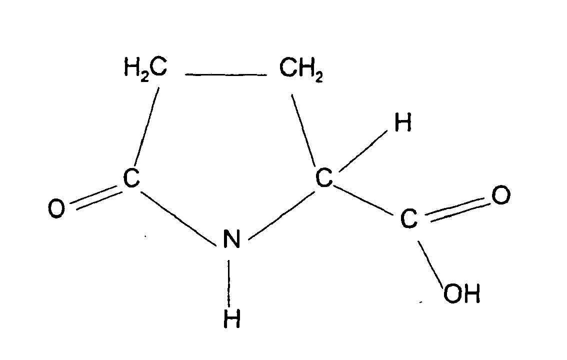

Other chitosan materials suitable for use herein include cross-linked chitosans with a low degree of cross-linkage and modified chitosans. Suitable crosslinking agents for use herein are organic compounds having at least two functional groups or functionalities capable of reacting with active groups located on the chitosan materials. Examples of such active groups include, but are not limited to, carboxylic acid (-COOH), amino (-NH₂), or hydroxyl (-OH) groups. Examples of such suitable crosslinking agents include, but are not limited to, diamines, polyamines, diols, polyols, dicarboxylic acids, polycarboxylic acids, aminocarboxylic acids, aminopolycarboxylic acids, polyoxides and the like. One way to introduce a crosslinking agent with the chitosan material solution is to mix the crosslinking agent with chitosan during preparation of the solution. Another suitable crosslinking agent comprises a metal ion with more than two positive charges, such as Ca²⁺, Al³⁺, Fe³⁺, Ce³⁺, Ce⁴⁺, Ti⁴⁺, Zr⁴+ and Cr³⁺. Since the cations on chitosan possess antimicrobial properties, it is preferred herein to not use a crosslinking agent reacting to the cations, unless no alternative crosslinking agent is available.

Modified chitosans for use herein are any chitosans where the glucan chains carry pendant groups. Examples of such modified chitosans include carboxymethyl chitosan, methyl pyrrolidinone chitosan, glycol chitosan and the like. Methyl pyrrolidone chitosan is for instance described in US 5,378,472. Water-soluble glycol chitosan and carboxymethyl chitosan are for instance described in WO 87/07618. Particularly suitable modified chitosans for use herein include water soluble covalently bonded chitosan derivatives or ionically bonded chitosan derivatives obtained by contacting salt of chitosan with electrophilic organic reagents. Such water-soluble chitosan derivatives are described in EP-A-737,692. Examples of chitosan derivatives suitable for use herein are described in depth in EP-A-737,692.

Suitable chitosan material is commercially available from numerous vendors. Exemplary of a commercially available chitosan materials are those available from for example the Vanson Company. The preferred chitosan salt for use herein is chitosan pyrrolidone carboxylate (also called chitosonium pyrrolidone carboxylate), which has a degree of deacetylation of more than 85%, a water solubility of 1% (1 gram is soluble in 100 grams of distilled water at 25°C and one atmosphere) and a pH of about 5. Chitosonium pyrrolidone carboxylate is commercially available under the name Kytamer® PC from Amerchol Corporation. Another preferred chitosan salt for use herein is chitosan lactate, the chitosan salt of lactic acid, which is commercially available from Vanson Company, Redmond, WA, USA.

By 'particles' as used herein refers to discrete flakes, fibres, beads and the like or mixtures thereof, of chitosan material. The term 'particles' herein also includes agglomerations or aggregations of discrete flakes, fibres, beads and the like of a certain material. 'Particle size' as used herein means the weighted average of the smallest dimension of the individual particles.

'Film-like region' as used herein refers to any area located on or within the absorbent member, which comprises particles of a chitosan material, wherein the individual particles are in such close contact to each other, such that a substantially continuous and homogenous layer of chitosan material is created. Such a film-like region of particles of chitosan material can be located on or inside of the absorbent member according to the present invention. In a film-like region said substantially continuous and homogenous layer of chitosan material not only covers concrete material, such as fibre surfaces or the walls of foam cells, but also spans across void spaces, i.e. interfibre spaces, pores, holes, apertures and the like, and thus forms a film-like pattern (see Figure 2). The thickness of said substantially continuous and homogenous layer of chitosan material typically is from 20 nm to 100 µm, preferably from 100 nm to 70 µm and more preferably from 1 µm to 20 µm. In a preferred embodiment herein, wherein the optionally applied latex underlies the chitosan material, the chitosan material covers not only the latex, which was applied onto the surface of the absorbent member prior to the application of the chitosan material, but also those parts of the absorbent member's surface, which do not comprise latex. It is understood herein that such a film-like region can be present in a fraction of the total absorbent member or might be present in the total absorbent member per se. For example in absorbent articles, especially those for feminine protection like sanitary napkins or panty liners, such absorbent members are used as absorbent cores and/or as secondary topsheets or secondary backsheets. In those applications, the absorbent members typically comprise film-like regions of the present invention in certain areas only. An example is the so-called central region, i.e., a region where body fluids like menstruation is discharged in use. Other examples are the in longitudinal or lateral zones, i.e., the peripheral edges of the absorbent articles, where run-off leakage of liquid needs to be prevented. Said film-like regions can have any size or shape. Said region can be substantially coextensive to one or both of the entire surfaces of the absorbent member or to only a fraction of said surfaces. Said regions can have an regular or irregular shape, including but not limited to, dots, squares, circles, ellipses, continuous or discontinuous stripes, and so on.

One reason for the outstanding leakage prevention benefits associated to the present invention is the presence of the chitosan material in film-like form. This achieves on the one hand a substantially continuous and homogeneous coverage of chitosan material in a certain region of the absorbent article and thus reliably immobilizes occurring free fluids in that region. On the other hand the film like application achieves, because of the close contact of the particles of chitosan material to each other, a layer, which is substantially impermeable towards body fluids.

In the instance that the film-like region is located on a surface of the absorbent member according to the present invention the area coverage of the chitosan material can be measured by the test method disclosed herein. By this, the ratio of the surface of the absorbent member in the film-like region, which is covered with chitosan material, can be determined. In a preferred embodiment the film-like region of chitosan material covers at least 75%, more preferably at least 80%, even more preferable at least 90% and most preferably up to 100% of the total surface of at least one surface of said absorbent member.

According to a preferred embodiment of the present invention at least 40%, preferably 60%, more preferably 80% and most preferably 100% of at least one surface of said absorbent member is covered by film-like regions comprising particles of chitosan material.

Typically, a film-like region of the absorbent member comprises particles of chitosan material at a level of from 0.1 g/m² to 200 g/m², preferably from 1 to 100 g/m², and more preferably from 2 to 50 g/m² of said absorbent member.

The film-like regions of the absorbent member according to the present invention comprise particles of chitosan material having a particle size distribution with a mean diameter D(v,0.9) of not more than 300 µm. According to the present invention these particles have a particle size distribution with a mean diameter D(v,0.9) of not from 10 nm to 300 µm, preferably from 20 nm to 100 µm and more preferably from 100 nm to 50 µm. By 'mean diameter D(v,x) of less than y µm' for a particle size distribution it is meant that (x*100)% of the particles have a mean diameter of less than y µm. For instance, a D(v,0.9) of not more than 100 µm indicates that 90% of the particles of chitosan material have a mean diameter of not more than 100 µm. The particle size distribution has been determined by the method as disclosed herein.

Because of the small size of the particles of chitosan material used herein, as defined by the selected particle size, the active surface area is very high compared to bigger particles for a same total weight. This contributes to the improved activity of the film-like application of particles of chitosan material according to the present invention. Advantageously, due to the high active surface area of the small chitosan material particles according to the present invention, the gelification and retention properties of chitosan material towards electrolyte-containing fluids are improved and hence the leakage prevention is significantly improved, this while using significantly less total amount of chitosan material.

It is to be understood herein that any method known in the art to provide an absorbent member with a film-like region comprising particles of chitosan material is suitable to be used herein. This includes spraying processes, curtain coating, printing and slot coating processes. Highly preferred herein is to use a spraying process as described in more details herein after in the process for making a preferred absorbent member according to the present invention.

The absorbent member according to the present invention can comprise further optional components. In a preferred embodiment, the absorbent member according to the present invention might comprise a latex binder. Typically, the absorbent member according to the present invention comprises from 1 to 30 g/m², preferably from 1 to 20 g/m² and more preferably from 1 to 10 g/m² of said absorbent member of latex.

In a particularly preferred embodiment, the absorbent member further contains particulate superabsorbent polymeric material, such as anionic superabsorbent material like absorbent gelling material based on polyacrylates. The superabsorbent polymeric material suitable for use herein can be in the form of fibres or of powder. Typically, the absorbent member according to the present invention comprises from 5 to 300 g/m², preferably from 20 to 150 g/m², and more preferably from 30 to 75 g/m² of said absorbent member of particulate superabsorbent material.

Another class of compounds to be optionally comprised by the absorbent member according to the present invention are odour control compounds. In particular, the absorbent member according to the present invention can comprise silica, zeolites, pH-adjusting material, chelants like EDTA, metal ions, cyclodextrins, urease inhibitors, antimicrobial compounds, activated carbon and mixtures thereof.

### Process for producing an absorbent member according to the present invention

The process for making the absorbent member according to the present invention is characterized by the essential steps of forming a precursor web, subsequently applying chitosan material onto at least one surface of the precursor web by a particular spray method and finally drying the resulting absorbent member.

The term 'precursor web' as used herein refers to absorbent materials, which serve as the basis for making the absorbent member according to the present invention. The precursor web for use herein typically has significant internal void space in the form of pores, holes, apertures, interstitial space between fibres and the like. Examples of precursor webs for use in the present invention are fibrous structures, such as nonwovens or fabrics, comprising natural or synthetic fibres or mixtures thereof, or apertured polymeric films or foam materials. Indeed, the precursor web for use in the present invention can be made of any of a variety of fibres, including a blend or admixture. The fibres may be cellulosic, modified cellulosic, or hydrophilic synthetic and include such fibres as wood pulp, rayon, cotton, cellulose acetate, polyester, nylon and the like. The precursor web can be made according to any suitable method known for this purpose in the art. Fibrous precursor webs according to the present invention can be made by appropriate processes such as dry laying and in particular air laying, melt blowing or spunbonding. Film-like or foam-like precursor webs according to the present invention are made by processes suitable for such purposes.

Highly preferred precursor webs for use herein are hydrophilic fibrous webs. As used herein, 'hydrophilic' refers to a material having a contact angle of water in air of less than 90 degrees, whereas the term 'hydrophobic' herein refers to a material having a contact angle of water in air of 90 degrees or greater. A precursor web comprising hydrophilic fibres like for example cellulosic fibres such as wood pulp fibres is particularly useful as an absorbent structure in products like sanitary napkins, disposable diapers or wipes because the hydrophilic fibres are liquid absorbent and therefore enhance the overall absorbency of the precursor web. Preferably, precursor webs for use herein can be made of a blend of cellulosic and hydrophilic synthetic fibres, typically comprising about 65% to 95% by weight of cellulosic fibres and more preferably up to about 20% by weight of the hydrophilic synthetic fibres. The hydrophilic synthetic fibres, which can be provided in any length including staple length, can improve the strength of the precursor web. Hydrophobic fibres or films, such as fibres or films made of polyethylene or polypropylene, may also be used in the precursor web herein provided they are treated by e.g. surfactants to make them hydrophilic, in order not to decrease the absorbent capacity of the preferred absorbent member, when incorporating them into said precursor web.

In a preferred embodiment the precursor web for use herein is a dry laid, preferably an air laid fibrous web. 'Dry laying' and more specifically, 'air laying' processes are widely used to produce webs from dry fibres, which can in turn be used e.g. as webs for absorbing fluids. Particularly, dry laying refers to e.g. carding or air laying. Carding refers to the formation of carded precursor webs, i.e. precursor webs in which the fibres are oriented (carded) in a given direction, whereas the air laying process refers to the formation of precursor webs with a completely random fibre orientation; the properties of such air laid precursor webs are therefore somewhat isotropic. The precursor webs produced by dry laying processes are soft, flexible and porous and are particularly suitable for use as liquid absorbent structures in absorbent articles, such as disposable diapers, sanitary napkins, incontinent pads and wipes.

The dry laid manufacturing process generally comprises a web formation and layering step and a web bonding and stabilizing step; in dry laying processes in fact the fibres, that can be of any type, e.g. cellulosic, synthetic, or any combination thereof, are formed or condensed into a web, but such web lacks integrity and must therefore be stabilized. Different techniques for bonding and stabilizing a dry formed web are known in the art, i.e. mechanical, thermal and chemical bonding processes. Bonding a web structure by means of a chemical agent is one of the most common methods of bonding in the nonwoven industry and consists in the application of a chemical binder to the web and in the curing of the binder.

Indeed, in the process according to the present invention the chitosan material is applied onto the precursor web as a solution or dispersion in the form of a spray of droplets having a droplet size distribution with a mean diameter D(v,0.9) of not more than 1500 µm, the amount of chitosan material solution or dispersion applied onto the precursor web being preferably from 1 ml to 1000 ml per square meter of said precursor web.

It has now been found that by applying the solution or dispersion of chitosan material on a precursor web in the form of a spray of droplets having a droplet size distribution with a mean diameter D(v,0.9) of not more than 1500 µm, preferably not more than 1000 µm, more preferably not more than 750 µm, a film-like region comprising particles of chitosan material is provided on the precursor web which translates in excellent fluid handling and gelifying performance, namely towards electrolyte-containing fluids, while requiring less chitosan material. Indeed, by applying the chitosan material solution or dispersion onto the precursor web in the form of a spray of small droplets as defined herein, a higher coverage of the surface sprayed is achieved, as compared to applying the same chitosan material solution or dispersion but in the form of a spray of droplets with larger droplets. Furthermore, applying the solution or dispersion of chitosan material on the precursor web as mentioned herein, translates into limited wetting of the surface, and thus in faster drying of the solution or dispersion of chitosan material. In other words, a film-like region comprising particles of chitosan material is generated in less time, resulting in improved processability and hence reduced process costs. A further benefit of applying the solution or dispersion defined infra as spray of small droplets is that the depth of penetration of said solution or dispersion is lower than the one of a spray of larger droplets. The advantage is that the precursor web is less wetted in its depth, which reduces the time for drying of said web after the chitosan application.

According to the above-described spraying procedure a solution or dispersion of chitosan material is provided on the precursor web, in which after drying a film-like region of particles of chitosan material is generated, wherein said particles have a particle size distribution with a mean diameter D(v,0.9) of not more than about 300 µm. According to the present invention these particles have a particle size distribution with a mean diameter D(v,0.9) of not from 10 nm to 300 µm, preferably from 20 nm to 100 µm and more preferably from 100 nm to 50 µm. Said particle size is a result of the selected droplet size of the spray of droplets as described above.

In Figure 3, the small particle size (namely less than 300 µm) of the adjacent particles of chitosan material, when applied according to the process of the present invention, and the homogeneity and continuity of the film-like pattern of the chitosan material particles on the surface of a fibrous precursor web is illustrated.

By 'mean diameter D(v,x) of not more than y µm' for a droplet size distribution it is meant that (x*100)% of the spray of droplets dispensed (expressed in volume unit) has a mean droplet diameter of not more than y µm. For instance, a D(v,0.9) of not more than 1500 µm indicates that 90% of the total sprayed volume is dispensed with droplets whose mean diameter is not more than 1500 µm. The droplet size distribution has been determined by the test method disclosed herein.

Any apparatus adapted to deliver a spray of droplets as defined herein are suitable for use herein. Several modifications can be made to the conventional, single aperture, spray head to ensure that a spray of such droplets as required herein is formed. Suitable apparatuses to be used herein (also called spray dispensers) share the common feature of having at least one aperture or a plurality of apertures also called "dispensing openings" or "spray nozzles" through which the solution/dispersion of the chitosan material is dispensed already mixed with air, said apertures being configured so as to deliver a spray of droplets having the characteristics mentioned herein. Suitable apparatus for use herein are air atomizers or nebulizators, which may be electrically operated.

In its most generic form, the process for making an absorbent member according to the present invention comprises the steps of:
(a) forming a precursor web having a first and a second surface, said second surface being approximately aligned opposite to said first surface, and
(b) applying during process step (a) onto at least one surface of said precursor web a solution or dispersion comprising a chitosan material, and/or
(b') applying after process step (a) onto at least one surface of said precursor web a solution or dispersion comprising a chitosan material,
   said solution or dispersion is applied onto said precursor web in the form of a spray of droplets as described herein, and
(c) drying said precursor web, whereby forming at least one film-like region comprising chitosan material on said surface of said precursor web on which said solution or dispersion of chitosan material was applied in steps (b) and/or (b').

In a preferred embodiment herein, the process comprises subsequently to step (a) the additional steps of applying latex onto at least one surface of said precursor web and drying said precursor web. After the drying, step (b') follows. Step (b) is not carried out in this embodiment.

Optionally, in cases where film-like regions of chitosan material are required between the surfaces of the absorbent member according to the present invention, an additional step (d) can be carried out after step (c). This additional step (d) is a second web forming process onto the surface of the precursor web, where the solution or dispersion of chitosan material was applied. This results in an absorbent member with at least one film-like region inside of it, i.e. between its surfaces. The second web forming step (d) can either be equal to the initial step of forming the precursor web (a) or different, which would result in a film-like region of chitosan material being located in a relatively homogeneous absorbent member or in an inhomogeneous absorbent member comprising two different web layers. However, similar structures can be achieved by placing a second already-formed web on said surface of the precursor web, where the solution or dispersion of chitosan material was applied.

Several methods are known for applying latex binder to the precursor web, while spray bonding and print bonding are particularly preferred herein. The 'latex' is usually an aqueous dispersion of a polymeric component and can be applied to a surface of the precursor web. Preferably, the polymeric component of the aqueous latex for use in the present invention substantially consists of hydrophilic material.

The latex is applied as an aqueous emulsion or dispersion, which typically contains about 5 to 65% and preferably, 10% of solids. These latex materials are readily available from several manufacturers. Because the latex dispersions are water miscible, they may be further diluted, if desired, before being applied to the precursor web. In addition, these latex compositions are thermosetting and in order to effect cross-linking, they can contain a small amount of suitable cross-linking agents which are well known chemical agents for this purpose, such as N-methylolacrylamide. Any type of latex known in the art can be used herein, if the polymeric component is substantially hydrophilic and the latex does not generate detectable odours, especially after curing, which would be unacceptable to the wearer. Latices available are classified by chemical family and those particularly useful herein include vinyl acetate and acrylic ester copolymers, ethylene vinyl acetate copolymers, styrene butadiene carboxylate copolymers and polyacrylonitriles and sold, for example, under the trade names of Airbond, Airflex and Vinac of Air Products, Inc., Hycar and Geon of Goodrich Chemical Co. and Fulatex of H. B. Fuller Company. A particularly preferred example of latex suitable for use in the present invention is Airflex 192, obtainable from Air Products and Chemicals Inc., Allentown, PA, USA. The amount of latex used in the absorbent member according to the present invention cannot be so high as to substantially impair or obscure the effective gelification capacity of the chitosan material and the absorbent properties of the hydrophilic fibres, or as to impart a degree of stiffness to the absorbent member as to render it impractical. The latex is applied onto the surface of the precursor web at a loading of from about 1 to 30 g/m², preferably from about 1 to 20 g/m² and more preferably from 1 to 10 g/m² of the precursor web. Latex with substantially hydrophobic polymeric components can also be used in the present invention by rendering them hydrophilic after their application onto the precursor web, e.g. by surfactants.

The presence of latex underlying the particles of chitosan material has the advantage of contributing to the control of the penetration of the chitosan particles into the precursor web to which they have been applied. Preferably, the chitosan particles do not penetrate into more than 30%, preferably not more than 20% and more preferably not more than 10% of the calliper of the precursor web. Because of this, the application of latex in the process of the present invention allows the production of particularly preferred absorbent members of the present invention that are even more effective in leakage prevention towards fluid while more efficiently using the chitosan material on the surface of the precursor web to which it is applied to. Indeed, it is speculated without wishing to be bound by theory that the application of latex provides a coating on the surface of the precursor web that reduces the depth of penetration of the solution or dispersion of chitosan material into the precursor web by partially sealing apertures, interfibre space and the like on said surface. The depth of penetration can easily be measured by cutting the absorbent member through its thickness and taking a picture of the cross-section of the cut absorbent under the microscope and subsequent evaluation of said picture.

In the following, an exemplary process is described for making the absorbent member according to the present invention. It should be understood that the process as described hereinafter is not limiting the scope of the present invention. It is outlined that the precursor web for use with the present invention can be made using conventional equipment designed for dry laying processes, although the following process is described with particular reference to air laid webs. It should be understood that other dry laying processes, e.g. carding, or other processes for creating fibrous substrates, such as the meltblown process or the spunbond process, or film forming or foaming are also applicable. It is also to be noted that besides the fact that in the following process the use of latex is described, this feature is optional and in no way limiting the scope of the present invention. The following reference numerals refer to Figure 1.

In accordance with a preferred embodiment of the present invention, the precursor web is made by an air laid process. The air forming system includes a distributor unit (1) disposed transversely above a continuous forming screen (3) mounted on rollers and driven by a suitable motor (not shown) and a vacuum means or suction box (2) is positioned beneath the screen (3). In a conventional air forming system, upstream of the distributor unit is a defibrator or feeder (not shown), such as a hammer mill or Rando-Feeder, where bales, laps or the like are defiberized and further the fibres may be cleaned and/or blended if necessary or desired depending largely on the type of fibres used, the blend of fibres used and the end product, namely the absorbent member, sought. For example, wood pulp fibres can be blended with substantially hydrophilic synthetic fibres and applied as a blend by a single distributor, or different fibres can be each conveyed by a different distributor to the screen to form separate plies or layers.

The porous forming screen (3) is essentially coextensive with the distributors (1), and the suction box (2) beneath the screen (3) draws the air stream downwardly and conveys the fibres to the surface of the screen (3), thereby forming a loose precursor web. At this stage of the process, the precursor web exhibits little integrity and the vacuum means (2) retains the loose, fibrous precursor web on the screen (3). The precursor web has a first surface that faces the distributor (1) and a second surface, opposite to said first surface, which faces the forming screen (3).

It should be understood that the system might be modified to control the composition and thickness of the end product, namely the absorbent member. For example, the distributor unit (1) can comprise a plurality of individual distributors and this number of distributors as well as their particular arrangement can be altered or varied depending on factors like machine speed, capacity, type of fibres and desired end product.

At this stage of the process, the precursor web on the screen (3) requires stabilization. The precursor web is advanced by the continuous screen and if desired, the precursor web first may be passed between compression rollers (not shown), which may be heated in order to increase the density of the precursor web, but this step is optional. This densification step also enhances the penetration of the latex, which is applied subsequently onto the precursor web and the degree or percentage of densification can vary depending on such factors like the basis weight of the precursor web, the desired degree of penetration of the latex into the precursor web and the desired end product.

From there, the precursor web is transported to the first latex application section (4) having a suitable dispensing means, such as a spray nozzle, doctor blade, roller applicator, or the like, where a liquid dispersion of the latex binder is applied to the first surface of the loose precursor web. Optionally, a vacuum applied by a suction box (5) positioned beneath the dispensing means and the screen helps to draw the latex dispersion into the precursor web. The dispensing means or applicator is essentially coextensive with the width of the precursor web and the latex is applied substantially homogeneously to the surface of the precursor web. However, the latex dispersion may be applied as a non-uniform or random application and because the latex dispersion is water-based, it will diffuse throughout the precursor web and function as a binder when cured.

The latex when cured imparts integrity to the precursor web and therefore some penetration of the latex is required. The extent or degree of penetration of the latex into the precursor web is controlled by the amount of latex applied and optionally by the vacuum applied to the precursor web in that the vacuum helps to draw the latex dispersion into the precursor web. The polymeric component of the latex is a substantially hydrophilic thermosetting plastic and in order to activate it, the latex dispersion can contain a suitable curing agent or cross-linking agent and after the latex is applied onto the precursor web, the latex is cured to effect cross-linking. In a particularly preferred embodiment, curing of the latex is accomplished by passing the latex-treated precursor web after the first latex application section (4) through a first drying section (6), e.g. a hot air oven or an air drier. The temperature inside of the first drying section typically ranges from about 100 °C to 260 °C, but this depends upon the specific type of latex resin used, upon the curing agent or cross-linking agent, upon the amount of latex, the thickness of the precursor web, the degree of vacuum and the machine speed. It is essential for the described process that the first surface of the latex-treated precursor web is substantially dry after the first drying section (6).

After the first drying section (6), the precursor web is being sprayed with the solution or dispersion of a chitosan material (preferably 4% by weight of chitosan material in water) onto the same surface, onto which latex was applied before in the chitosan application section (7). According to the present invention, the solution or dispersion of the chitosan material is applied on the precursor web in the form of a spray of droplets having a droplet size distribution with a mean diameter D(v,0.9) of not more than 1500 µm. The amount of chitosan material solution or dispersion applied onto the precursor web is preferably from 1 ml to 1000 ml, more preferably from 1 ml to 400 ml and most preferably about 120 ml of solution or dispersion of chitosan material per square meter of precursor web. For achieving the above-mentioned particle size distribution of said spray, it is particularly preferred to use so-called air atomizers or nebulizators for applying the solution of the chitosan material onto the surface of the precursor web. Examples therefore are the air atomising nozzles of the ¼ JAU series from Spraying Systems, Co., Wheaton, Illinois, USA. By such a spray process, after drying a film-like region of chitosan material particles having a particle size distribution with a mean diameter D(v,0.9) of not more than 300 µm are generated on the surface of the precursor web, which was sprayed with the solution or dispersion of the chitosan material.

It is preferred to also apply latex to the second surface of the precursor web as well. Therefore, the precursor web is preferably reverted. After the reversion step, latex is applied onto the second surface of the precursor web by the second latex application section (8) in essentially the same way as the first surface in the first latex application section (4). In addition, the second latex application section (8) can include a suction box (9) for controlling the penetration of the latex into the precursor web. This second latex application is likewise cured by passing the precursor web through a second drying section (10) subsequent to the second latex application section (8) within about the same temperature range as indicated at the first drying section (6).

The absorbent member resulting from the above process passes, after having left the second drying section (10), a subsequent third drying section (11) for removing last traces of moisture within about the same temperature range as indicated at the first drying section (6). Afterwards the absorbent member exhibits sufficient integrity and can be cut, rolled, packaged, etc.

In an alternative embodiment, the chitosan material can be sprayed onto the second surface of the precursor web after the second drying section (10), instead onto the first surface after the first drying section (6). For this purpose, the second surface of the precursor web has to be substantially dry after having passed the second drying section (10).

It is also within the scope of the present invention that the chitosan material is applied onto the first and/or second surface of the precursor web after the first (4) or the second (8) latex application sections, when the precursor web is still wet from the latex dispersion applied.

The concentration of the solution or dispersion of chitosan material to be applied onto the precursor web can vary from 0.1 to 40% by weight of chitosan material and is preferably from 1 to 10% by weight and more preferably 4% by weight of chitosan material. The pH of the solution or dispersion of chitosan material to be applied onto the precursor web is from 3 to 7, preferably from 4 to 6 and more preferably about 5 for an optimum match between skin-friendliness and water-solubility.

Notwithstanding the application of the latex, the absorbent member is soft yet strong and absorbent, exhibiting a relatively high tensile strength. It is desirable for preferred absorbent members of this type to have relatively low bulk, because a more dense absorbent member, when compared to similar absorbent members containing no latex and of about equal absorptive capacity but of higher bulk, can be thinner yet highly absorbent and consequently less bulky. A reduction in bulk, which means a reduction in volume the absorbent member is occupying, without sacrificing significantly other desired properties, is important from the standpoint of manufacturing, storage and packaging. Hence, for absorbent members of this invention the basis weight ranges from about 20 g/m² to 500 g/m², preferably from about 75 g/m² to 400 g/m² and more preferably from 100 g/m² to 200 g/m². There can be manufacturing constraints in producing an absorbent member having a basis weight lower than about 20 g/m² in that such an absorbent member may lack desired strength. When the basis weight exceeds the upper limit, the absorbent member may be too stiff and therefore not useful for most applications.

Absorbent members made according to the above-described process exhibit a good integrity due to the application of latex, combined with good liquid barrier capabilities. The depth of penetration of the latex binder into the precursor web is controlled by the choice of the polymer concentration and the amount of the latex to be applied onto the precursor web and optionally by the vacuum applied by means of the suction boxes positioned in correspondence with the dispensing means.

In an alternative embodiment of the absorbent member according to the present invention a porous reinforcing sheet such as a creped paper, a tissue, or a nonwoven, can be incorporated into the precursor web either as a surface sheet or as an intermediate sheet disposed intermediate the first and second surfaces of the absorbent member. The sheet can be present on one surface of the absorbent member while the opposite surface bears cured latex.

In another embodiment the precursor web can comprise a layer of polyester or polyolefin nonwoven having a layer of air laid fibres on top. Bonding between the fibres of the two layers is achieved by means of mechanical entangling, while the latex binder is subsequently only applied to the surface of the precursor web which is opposite to the polyester or polyolefin layer.

As mentioned before, the use of a binder, such as latex, is optional in the process of the present invention. Therefore, the above-described embodiment of the process for making the absorbent member is in no way limiting the scope of the present invention.

### Examples

### Example 1:

The absorbent member according to the present invention is illustrated by the following example: The absorbent member comprises an airlaid precursor web made of 68% of cellulose fibres (NB 416 by Weyerhaeuser) mixed with 17% of polyacrylic superabsorbent powder (Acqualic L74 by Nippon Shokubai), 11% of synthetic fibres (T255 3DTEX-3mm by Trevira), and 4% of latex (Elite 33 by Vinamul) (2% per surface), which was applied via a spraying system onto both surfaces of the absorbent member. Chitosan pyrrolidone carboxylate (Kytamer by Amerchol) was sprayed as a solution of 4% by weight of chitosan pyrrolidone carboxylate in water onto one entire surface of the airlaid precursor web at a loading of 6 g of chitosan pyrrolidone carboxylate per square meter of the absorbent member after drying (Air atomising system by Spraying Systems Corp., droplet size distribution with a mean diameter D(v,0.9) of from 5 to 400 nm). The resulting film-like region of particles of chitosan pyrrolidone carboxylate had an area coverage of about 90 % within said region; the chitosan pyrrolidone carboxylate particles had a particle size distribution with a mean diameter D(v,0.9) from 20 to 100 nm after drying. This absorbent member has been used for taking the pictures of figures 2 and 3.

### Example 2:

The absorbent member of the present invention is further illustrated by the following example: The absorbent member comprises an airlaid precursor web made of 68% of cellulose fibres (NB 416 from Weyerhauser) mixed with 17% of polyacrylic superabsorbent powder (Acqualic L74 from Nippon Shokubai), 11% of synthetic fibres (T255 3DTEX-3mm from Trevira), and 4% of latex (Elite 33 from Vinamul) (2% per surface), which was applied via a spraying system onto both surfaces of the absorbent member. Chitosan lactate (Chitosolv L from Vanson, Inc.) was sprayed as a solution of 6 % by weight of chitosan lactate in water onto one side of the airlaid precursor web at a loading of 6 g of Chitosan Lactate per square meter of the absorbent member after drying (Air atomising system from Spraying Systems Co., droplet size distribution with a mean diameter D(v,0.9) of from 5 to 400 nm). The resulting film-like region of particles of chitosan lactate had an area coverage than within said region of 90 %, the chitosan lactate particles had a particle size distribution with a mean diameter D(v,0.9) from 20 to 100 nm after drying.

### Test methods

### 1. Particle size distribution

The particle size distribution of the chitosan material can be determined by ESEM analysis, using a Philips XL30 ESEM FEG electronic microscope for example. A random sample of 1.5 cm x 1.5 cm was cut with a scissors from the part of the absorbent member comprising the film-like region of chitosan material and mounted on a circular aluminium stub having a diameter of 1.2 cm. The sample was then sputtered with a layer of gold having a thickness of 30 nm. The sample was observed in SEM mode *in vacuo* at an appropriate magnification to visually investigate the particle size of the chitosan material, taking six images in different zones of the sample. The size of the individual particles is determined visually.

### 2. Droplet size distribution

The droplet size distribution of a spray of droplets can be determined as follows: Suitable test equipment is for instance a Malvern Mastersizer S LongBed® with 1000 mm lens and a maximum particle size range of 3475 µm. The Malvem Mastersizer S LongBed® provides 21 cm opening (between lenses) to accommodate spray flow. In all readings at the Malvern® , the lens surface must remain free of spray contamination. In the present setup procedure, the distance from nozzle to laser was fixed at 8 cm to minimize lens contamination. At 8 cm distance, the spray was directed to the laser beam to place the laser centre to the spray cone. At least three readings have to be made for each sample of chitosan material solution/dispersion sprayed to determine the droplet size distribution of the spray of droplets. The sprayer used in this test was an electrically operated sprayer.

### 3. Area coverage assessment

The area coverage assessment is made by using ESEM analysis (using a Philips XL30 ESEM FEG electronic microscope for example) coupled with a SIS elaboration system (Soft Imaging System). Three different areas of a film-like region the absorbent member have been selected in a random way. The samples were cut to diameters of about 1.2 cm with a scissors from these areas and mounted on a circular aluminium stub having a diameter of 1.2 cm, and then sputtered with a layer of gold having a thickness of 30 nm. The sample was observed in SEM mode *in vacuo* at an appropriate magnification (preferably 30X). For each samples taken 6 images were taken. The evaluation of uncovered area has been made by elaborating the different pictures with the SIS Image Analysis (based on detection of different colour intensity). Determination of the final percentage of coverage has been made by a proportion at 100% of the obtained values in µm².

## Claims

1. A disposable absorbent article comprising:
a liquid pervious topsheet;
a liquid impervious backsheet; and
an absorbent member positioned between said topsheet and said backsheet, said absorbent member having a thickness dimension, a first surface being oriented towards said topsheet and an opposed second surface being oriented towards said backsheet, said second surface being separated from said first surface by said thickness dimension,
said article being **characterized in that** said absorbent member comprises at least one film-like region of particles of chitosan material, said particles having a particle size distribution with a mean diameter D(v,0.9) of not more than 300 µm.

2. The absorbent article of claim 1, **characterized in that** said film-like region is positioned on said first and/or second surface of said absorbent member, preferably said second surface.

3. The absorbent article of claim 2, **characterized in that** the surface area coverage of chitosan material within said film-like region of particles of chitosan material on said surface of said absorbent member is at least 75%, preferably at least 80%, even more preferably at least 90% and most preferably about 100% of the total surface of said film-like region.

4. The absorbent article of any of the preceding claims, **characterized in that** said article further comprises an additional absorbent member positioned between said film-like region and said backsheet and/or said topsheet.

5. The absorbent article of any of the preceding claims, **characterized in that** said film-like region comprises chitosan particles having a particle size distribution with a mean diameter D(v,0.9) of from 10 nm to 300 µm, preferably from 20 nm to 100 µm and more preferably from 100 nm to 50 µm.

6. The absorbent article of any of the preceding claims, **characterized in that** said chitosan material has a degree of deacetylation of more than 70%, preferably from 80% to 100%.

7. The absorbent article of any of the preceding claims, **characterized in that** said chitosan material comprises at least one salt of chitosan, said salt preferably being chitosonium pyrrolidone carboxylate and/or chitosonium lactate.

8. The absorbent article of any of the preceding claims, **characterized in that** said absorbent member has internal void space and preferably comprises a dry laid, more preferably an air laid, hydrophilic fibrous web.

9. The absorbent article of any of the preceding claims, **characterized in that** said film-like region comprises particles of chitosan material in an amount of 0.1 to 200 g per square meter, preferably from 1 to 100 g per square meter and more preferably from 2 to 50 g per square meter of said absorbent member.

10. The absorbent article of any of the preceding claims, **characterized in that** at least one of said surfaces of said absorbent member is covered by at least 40%, preferably 60%, more preferably 80% and most preferably 100% of the total surface area of said surface with said film-like regions comprising particles of chitosan material.

11. Process for making an absorbent member, said process comprising the steps of:
(a) forming a precursor web having a first and a second surface, said second surface being approximately aligned opposite to said first surface, and
(b) applying during process step (a) onto at least one surface of said precursor web a solution or dispersion comprising a chitosan material, and/or
(b') applying after process step (a) onto at least one surface of said precursor web a solution or dispersion comprising a chitosan material, and
(c) drying said precursor web, whereby forming at least one film-like region comprising particles of chitosan material on said surface of said precursor web on which said solution or dispersion of chitosan material was applied in steps (b) and/or (b'),
said process being **characterized in that** said solution or dispersion is applied onto said precursor web in the form of a spray of droplets, said droplets having a droplet size distribution with a mean diameter D(v,0.9) of not more than 1500 µm.

12. The process of claim 11, **characterized in that** said droplets having a droplet size distribution with a mean diameter D(v,0.9) not more than 1000 µm, preferably not more than 750 µm.

13. The process of claims 11 or 12, **characterized in that** the surface area coverage of chitosan material within said film-like region of particles of chitosan material on said surface of said precursor web is at least 75%, preferably at least 80%, even more preferably at least 90% and most preferably about 100% of the total surface of said film-like region.

14. The process of claims 11-13, wherein step (b) is not carried out, said process comprising the additional steps of:
(a') applying latex onto at least one surface of said precursor web, and
(a") drying said precursor web, said process being **characterized in that** steps (a') and (a") are carried out after said step (a) and before said step (b').

15. The process of any of claims 11 - 14, comprising the additional step of:
(d) second web formation process, **characterized in that** step (d) is carried out after said step (c).

16. The process of any of claims 11 - 15, **characterized in that** said precursor web has internal void space, preferably being a dry laid, more preferably an air laid, hydrophilic fibrous web.

17. The process of any of claims 11 - 16, **characterized in that** said absorbent member comprises after step (c) particles of chitosan material having a particle size distribution with a mean diameter D(v,0.9) of from 10 nm to 300 µm, preferably from 20 nm to 100 µm and more preferably from 100 nm to 50 µm.

18. The process of any of claims 11 - 17, **characterized in that** said chitosan material penetrates into said precursor web to not more than 30%, preferably not more than 20% and more preferably not more than 10% by calliper of said precursor web.

19. The process of any of claims 14 - 18, **characterized in that** said latex is applied to said surface of said precursor web at a loading of from 1 to 30 g/m², preferably from 1 to 20 g/m² and more preferably from 1 to 10 g/m².

20. The process of any of claims 11 - 19, **characterized in that** said chitosan material comprises at least one substantially water-soluble salt of chitosan, said salt preferably being chitosonium pyrrolidone carboxylate and/or chitosonium lactate.

21. The process of any of claims 11 - 20, **characterized in that** said solution or dispersion of chitosan material is an aqueous solution or dispersion, comprising from 0.1 to 40%, preferably from 1 to 10% and more preferably about 4% by weight of said chitosan material.

22. The process of any of claims 11 - 21, **characterized in that** said solution or dispersion of chitosan material is applied in an amount of 1 to 1000 ml, preferably from 1 to 400 ml and more preferably from 1 to 150 ml of said solution or dispersion of chitosan material per square meter of said precursor web.

23. The process of any of claims 11 - 22, **characterized in that** said solution or dispersion of chitosan material is applied onto at least one surface of said precursor web across at least 40%, preferably 60%, more preferably 80% and most preferably 100% of the whole surface of said precursor web.

24. Absorbent article comprising a liquid-pervious topsheet, a liquid-impervious backsheet and an absorbent core, said absorbent core comprising an absorbent member made according to any of claim 11-23.

25. The absorbent article of any of claims 1-10 or 24, **characterized in that** said liquid-impervious backsheet is a breathable backsheet allowing transfer of air and/or water vapour therethrough.

26. The absorbent article of any of claims 1-10, 24 or 25, **characterized in that** said absorbent article is an absorbent article for feminine hygiene.

## Patentansprüche

1. Einwegabsorptionsartikel, umfassend:
eine flüssigkeitsdurchlässige obere Lage;
eine flüssigkeitsundurchlässige untere Lage; und
ein absorbierendes Element, das sich zwischen der oberen Lage und der unteren Lage befindet, wobei das absorbierende Element eine Dickenabmessung, eine erste Oberfläche, die zu der oberen Lage ausgerichtet ist, und eine gegenüber liegende zweite Oberfläche, die zu der unteren Lage ausgerichtet ist, aufweist, wobei die zweite Oberfläche durch die Dickenabmessung von der ersten Oberfläche getrennt ist,
wobei der Artikel **dadurch gekennzeichnet ist, dass** das absorbierende Element mindestens einen filmartigen Bereich von Teilchen aus Chitosanmaterial umfasst, wobei die Teilchen eine Teilchengrößenverteilung mit einem mittleren Durchmesser D(v,0,9) von nicht mehr als 300 µm aufweisen

2. Absorptionsartikel nach Anspruch 1, **dadurch gekennzeichnet, dass** der filmartige Bereich auf der ersten und/oder zweiten Oberfläche des absorbierenden Elements, vorzugsweise auf der zweiten Oberfläche, angeordnet ist.

3. Absorptionsartikel nach Anspruch 2, **dadurch gekennzeichnet, dass** die Oberflächenabdeckung mit Chitosanmaterial innerhalb des filmartigen Bereichs von Teilchen aus Chitosanmaterial auf der Oberfläche des absorbierenden Elements mindestens 75 %, vorzugsweise mindestens 80 %, noch mehr bevorzugt mindestens 90 % und am meisten bevorzugt ungefähr 100 % der Gesamtfläche des filmartigen Bereichs beträgt.

4. Absorptionsartikel nach einem der vorstehenden Ansprüche, **dadurch gekennzeichnet, dass** der Artikel ferner ein zusätzliches absorbierendes Element, das sich zwischen dem filmartigen Bereich und der unteren Lage und/oder der oberen Lage befindet, umfasst.

5. Absorptionsartikel nach einem der vorstehenden Ansprüche, **dadurch gekennzeichnet, dass** der filmartige Bereich Chitosanteilchen mit einer Teilchengrößenverteilung mit einem mittleren Durchmesser D(v,0,9) von 10 nm bis 300 µm, vorzugsweise von 20 nm bis 100 µm und mehr bevorzugt von 100 nm bis 50 µm umfasst.

6. Absorptionsartikel nach einem der vorstehenden Ansprüche, **dadurch gekennzeichnet, dass** das Chitosanmaterial einen Deacetylierungsgrad von mehr als 70 %, vorzugsweise von 80 % bis 100 % aufweist.

7. Absorptionsartikel nach einem der vorstehenden Ansprüche, **dadurch gekennzeichnet, dass** das Chitosanmaterial mindestens ein Chitosansalz umfasst, wobei das Salz vorzugsweise Chitosoniumpyrrolidoncarboxylat und/oder Chitosoniumlactat ist.

8. Absorptionsartikel nach einem der vorstehenden Ansprüche, **dadurch gekennzeichnet, dass** das absorbierende Element einen Innenhohlraum aufweist und vorzugsweise eine trockengelegte, mehr bevorzugt eine luftgelegte, hydrophile Faserbahn umfasst.

9. Absorptionsartikel nach einem der vorstehenden Ansprüche, **dadurch gekennzeichnet, dass** der filmartige Bereich Teilchen aus Chitosanmaterial in einer Menge von 0,1 bis 200 g pro Quadratmeter, vorzugsweise von 1 bis 100 g pro Quadratmeter und mehr bevorzugt von 2 bis 50 g pro Quadratmeter des absorbierenden Elements umfasst.

10. Absorptionsartikel nach einem der vorstehenden Ansprüche, **dadurch gekennzeichnet, dass** mindestens eine der Oberflächen des absorbierenden Elements zu mindestens 40 %, vorzugsweise 60 %, mehr bevorzugt 80 % und am meisten bevorzugt 100 % der Gesamtfläche der Oberfläche mit den filmartigen Bereichen, die Teilchen aus Chitosanmaterial umfassen, bedeckt ist.

11. Verfahren zum Herstellen eines absorbierenden Elements, wobei das Verfahren die folgenden Schritte umfasst:
(a) Bilden einer Vorläuferbahn, die eine erste und eine zweite Oberfläche aufweist, wobei die zweite Oberfläche gegenüber der ersten Oberfläche ungefähr bündig ausgerichtet ist, und
(b) Auftragen einer Lösung oder Dispersion, die ein Chitosanmaterial umfasst, auf mindestens eine Oberfläche der Vorläuferbahn während Verfahrensschritt (a), und/oder
(b) Auftragen einer Lösung oder Dispersion, die ein Chitosanmaterial umfasst, auf mindestens eine Oberfläche der Vorläuferbahn nach Verfahrensschritt (a), und
(c) Trocknen der Vorläuferbahn, dadurch Bilden mindestens eines filmartigen Bereichs, der Teilchen aus Chitosanmaterial umfasst, auf der Oberfläche der Vorläuferbahn, auf die die Lösung oder Dispersion von Chitosanmaterial in den Schritten (b) und/oder (b') aufgetragen wurde,
wobei das Verfahren **dadurch gekennzeichnet ist, dass** die Lösung oder Dispersion in der Form eines Sprays oder von Tröpfchen auf die Vorläuferbahn aufgetragen wird, wobei die Tröpfchen eine Tröpfchengrößenverteilung mit einem mittleren Durchmesser D(v,0,9) von nicht mehr als 1500 µm aufweist.

12. Verfahren nach Anspruch 11, **dadurch gekennzeichnet, dass** die Tröpfchen eine Tröpfchengrößenverteilung mit einem mittleren Durchmesser D(v,0,9) von nicht mehr als 1000 µm, vorzugsweise nicht mehr als 750 µm aufweisen.

13. Verfahren nach Anspruch 11 oder 12, **dadurch gekennzeichnet, dass** die Oberflächenabdeckung mit Chitosanmaterial innerhalb des filmartigen Bereichs von Teilchen aus Chitosanmaterial auf der Oberfläche der Vorläuferbahn mindestens 75 %, vorzugsweise mindestens 80 %, noch mehr bevorzugt mindestens 90 % und am meisten bevorzugt ungefähr 100 % der Gesamtfläche des filmartigen Bereichs beträgt.

14. Verfahren nach Ansprüchen 11-13, wobei Schritt (b) nicht durchgeführt wird, wobei das Verfahren die folgenden zusätzlichen Schritte umfasst:
(a') Auftragen von Latex auf mindestens eine Oberfläche der Vorläuferbahn und
(a") Trocknen der Vorläuferbahn, wobei das Verfahren **dadurch gekennzeichnet ist, dass** die Schritte (a') und (a") nach dem Schritt (a) und vor dem Schritt (b') durchgeführt werden.

15. Verfahren nach einem der Ansprüche 11 - 14, das den folgenden zusätzlichen Schritt umfasst:
(d) Verfahren zur Bildung einer zweiten Bahn, **dadurch gekennzeichnet, dass** Schritt (d) nach dem Schritt (c) durchgeführt wird.

16. Verfahren nach einem der Ansprüche 11 - 15, **dadurch gekennzeichnet, dass** die Vorläuferbahn Innenhohlraum aufweist und vorzugsweise eine trockengelegte, mehr bevorzugt eine luftgelegte, hydrophile Faserbahn ist.

17. Verfahren nach einem der Ansprüche 11 - 16, **dadurch gekennzeichnet, dass** das absorbierende Element nach Schritt (c) Teilchen aus Chitosanmaterial mit einer Teilchengrößenverteilung mit einem mittleren Durchmesser D(v,0,9) von 10 nm bis 300 µm, vorzugsweise von 20 nm bis 100 µm und mehr bevorzugt von 100 nm bis 50 µm umfasst.

18. Verfahren nach einem der Ansprüche 11 - 17, **dadurch gekennzeichnet, dass** das Chitosanmaterial zu nicht mehr als 30 %, vorzugsweise nicht mehr als 20 % und mehr bevorzugt nicht mehr als 10 % der Dicke der Vorläuferbahn in die Vorläuferbahn eindringt.

19. Verfahren nach einem der Ansprüche 14 - 18, **dadurch gekennzeichnet, dass** der Latex in einer Stärke von 1 bis 30 g/m², vorzugsweise von 1 bis 20 g/m² und mehr bevorzugt von 1 bis 10 g/m² auf die Oberfläche der Vorläuferbahn aufgetragen wird.

20. Verfahren nach einem der Ansprüche 11 - 19, **dadurch gekennzeichnet, dass** das Chitosanmaterial mindestens ein im Wesentlichen wasserlösliches Chitosansalz umfasst, wobei das Salz vorzugsweise Chitosoniumpyrrolidoncarboxylat und/oder Chitosoniumlactat ist.

21. Verfahren nach einem der Ansprüche 11 - 20, **dadurch gekennzeichnet, dass** die Lösung oder Dispersion von Chitosanmaterial eine wässrige Lösung oder Dispersion ist, die von 0,1 bis 40 Gew.-%, vorzugsweise von 1 bis 10 Gew.-% und mehr bevorzugt ungefähr 4 Gew.-% das Chitosanmaterial umfasst.

22. Verfahren nach einem der Ansprüche 11 - 21, **dadurch gekennzeichnet, dass** die Lösung oder Dispersion von Chitosanmaterial in einer Menge von 1 bis 1000 ml, vorzugsweise von 1 bis 400 ml und mehr bevorzugt von 1 bis 150 ml Lösung oder Dispersion von Chitosanmaterial pro Quadratmeter der Vorläuferbahn aufgetragen wird.

23. Verfahren nach einem der Ansprüche 11 - 22, **dadurch gekennzeichnet, dass** die Lösung oder Dispersion von Chitosanmaterial auf mindestens eine Oberfläche der Vorläuferbahn über mindestens 40 %, vorzugsweise 60 %, mehr bevorzugt 80 % und am meisten bevorzugt 100 % der gesamten Oberfläche der Vorläuferbahn aufgetragen wird.

24. Absorptionsartikel, umfassend eine flüssigkeitsdurchlässige obere Lage, eine flüssigkeitsundurchlässige untere Lage und einen Absorptionskern, wobei der Absorptionskem ein absorbierendes Element, das nach einem der Ansprüche 11 - 23 hergestellt ist, umfasst.

25. Absorptionsartikel nach einem der Ansprüche 1 - 10 oder 24, **dadurch gekennzeichnet, dass** die flüssigkeitsundurchlässige untere Lage eine atmungsaktive untere Lage ist, die den Durchgang von Luft und/oder Wasserdampf dort hindurch ermöglicht.

26. Absorptionsartikel nach einem der Ansprüche 1 - 10, 24 oder 25, **dadurch gekennzeichnet, dass** der Absorptionsartikel ein Absorptionsartikel für die Damenhygiene ist.

## Revendications

1. Article absorbant jetable comprenant:
une feuille de dessus perméable aux liquides;
une feuille de fond imperméable aux liquides; et
un élément absorbant positionné entre ladite feuille de dessus et ladite feuille de fond, ledit élément absorbant ayant une dimension en épaisseur, une première surface étant orientée vers ladite feuille de dessus et une deuxième surface opposée orientée vers ladite feuille de fond, ladite deuxième surface étant séparée de ladite première surface par ladite dimension en épaisseur,
ledit article étant **caractérisé en ce que** ledit élément absorbant comprend au moins une région de type pellicule de particules de matériau de chitosan, lesdites particules ayant une granulométrie avec un diamètre moyen D(v,0,9) de pas plus de 300 µm.

2. Article absorbant selon la revendication 1, **caractérisé en ce que** ladite région de type pellicule est positionnée sur ladite première et/ou deuxième surface dudit élément absorbant, de préférence ladite deuxième surface.

3. Article absorbant selon la revendication 2, **caractérisé en ce que** la couverture superficielle de matériau de chitosan au sein de ladite région de type pellicule de particules de matériau de chitosan sur ladite surface dudit élément absorbant est au moins 75 %, de préférence au moins 80 %, encore plus préférablement au moins 90 % et le plus préférablement environ 100 % de la surface totale de ladite région de type pellicule.

4. Article absorbant selon l'une quelconque des revendications précédentes, **caractérisé en ce que** ledit article comprend, en outre, un élément absorbant supplémentaire positionné entre ladite région de type pellicule et ladite feuille de fond et/ou ladite feuille de dessus.

5. Article absorbant selon l'une quelconque des revendications précédentes, **caractérisé en ce que** ladite région de type pellicule comprend des particules de chitosan ayant une granulométrie avec un diamètre moyen D(v,0.9) allant de 10 nm à 300 µm, de préférence de 20 nm à 100 µm et plus préférablement de 100 nm à 50 µm.

6. Article absorbant selon l'une quelconque des revendications précédentes, **caractérisé en ce que** ledit matériau de chitosan a un degré de désacétylation de plus de 70 %, de préférence de 80 % à 100 %.

7. Article absorbant selon l'une quelconque des revendications précédentes, **caractérisé en ce que** ledit matériau de chitosan comprend au moins un sel de chitosan, ledit sel étant de préférence du pyrrolidone-carboxylate de chitosonium et/ou du lactate de chitosonium.

8. Article absorbant selon l'une quelconque des revendications précédentes, **caractérisé en ce que** ledit élément absorbant a un espace vide interne et comprend de préférence une nappe fibreuse hydrophile par voie sèche, plus préférablement appliquée par jet d'air.

9. Article absorbant selon l'une quelconque des revendications précédentes, **caractérisé en ce que** ladite région de type pellicule comprend des particules de matériau de chitosan en une quantité de 0,1 à 200 g par mètre carré, de préférence de 1 à 100 g par mètre carré et plus préférablement de 2 à 50 g par mètre carré dudit élément absorbant.

10. Article absorbant selon l'une quelconque des revendications précédentes, **caractérisé en ce qu'**au moins une desdites surfaces dudit élément absorbant est couverte par au moins 40 %, de préférence 60 %, plus préférablement 80 % et le plus préférablement 100 % de la superficie totale de ladite surface avec lesdites régions de type pellicule comprenant des particules de matériau de chitosan.

11. Procédé de fabrication d'un élément absorbant, ledit procédé comprenant les étapes consistant à:
(a) former une nappe de précurseur ayant une première et une deuxième surfaces, ladite deuxième surface étant approximativement alignée à l'opposé de ladite première surface, et
(b) appliquer durant une étape de procédé (a) sur au moins une surface de ladite nappe de précurseur une solution ou dispersion comprenant un matériau de chitosan, et/ou
(b') appliquer après une étape de procédé (a) sur au moins une surface de ladite nappe de précurseur une solution ou dispersion comprenant un matériau de chitosan, et/ou
(c) sécher ladite nappe de précurseur, selon quoi on forme au moins une région de type pellicule comprenant des particules de matériau de chitosan sur ladite surface de ladite nappe de précurseur sur laquelle ladite solution ou dispersion de matériau de chitosan avait été appliquée dans les étapes (b) et/ou (b'),
ledit procédé étant **caractérisé en ce que** ladite solution ou dispersion est appliquée sur ladite nappe de précurseur sous la forme d'un aérosol de gouttelettes, lesdites gouttelettes ayant une répartition granulométrique de gouttelettes avec un diamètre moyen D(v,0.9) de pas plus de 1500 µm.

12. Procédé selon la revendication 11, **caractérisé en ce que** lesdites gouttelettes ayant une répartition granulométrique de gouttelettes avec un diamètre moyen D(v,0.9) de pas plus de 1000 µm, de préférence pas plus de 750 µm.

13. Procédé selon les revendications 11 ou 12, **caractérisé en ce que** la couverture superficielle de matériau de chitosan au sein de ladite région de type pellicule de particules de matériau de chitosan sur ladite surface de ladite nappe de précurseur est au moins 75 %, de préférence au moins 80 %, encore plus préférablement au moins 90 % et le plus préférablement environ 100 % de la surface totale de ladite région de type pellicule.

14. Procédé selon les revendications 11 à 13, dans lequel l'étape (b) n'est pas effectuée, ledit procédé comprenant les étapes supplémentaires consistant à:
(a') appliquer un latex sur au moins une surface de ladite nappe de précurseur, et
(a") sécher ladite nappe de précurseur, ledit procédé étant **caractérisé en ce que** les étapes (a') et (a") sont effectuées après ladite étape (a) et avant ladite étape (b').

15. Procédé selon l'une quelconque des revendications 11 à 14, comprenant l'étape supplémentaire consistant en:
(d) un procédé de formation de deuxième nappe, **caractérisé en ce que** l'étape (d) est effectuée après ladite étape (c).

16. Procédé selon l'une quelconque des revendications 11 à 15, **caractérisé en ce que** ladite nappe de précurseur a un espace vide interne, étant de préférence une nappe fibreuse hydrophile par voie sèche, plus préférablement appliquée par jet d'air.

17. Procédé selon l'une quelconque des revendications 11 à 16, **caractérisé en ce que** ledit élément absorbant comprend après l'étape (c) des particules de matériau de chitosan ayant une granulométrie avec un diamètre moyen D(v,0,9) allant de 10 nm à 300 µm, de préférence de 20 nm à 100 µm et plus préférablement de 100 nm à 50 µm.

18. Procédé selon l'une quelconque des revendications 11 à 17, **caractérisé en ce que** ledit matériau de chitosan pénètre dans ladite nappe de précurseur à pas plus de 30 %, de préférence pas plus de 20 % et plus préférablement pas plus de 10 % par calibre de ladite nappe de précurseur.

19. Procédé selon l'une quelconque des revendications 14 à 18, **caractérisé en ce que** ledit latex est appliqué sur ladite surface de ladite nappe de précurseur à un chargement allant de 1 à 30 g/m², de préférence de 1 à 20 g/m² et plus préférablement de 1 à 10 g/m².

20. Procédé selon l'une quelconque des revendications 11 à 19, **caractérisé en ce que** ledit matériau de chitosan comprend au moins un sel sensiblement hydrosoluble de chitosan, ledit sel étant de préférence du pyrrolidone-carboxylate de chitosonium et/ou du lactate de chitosonium.

21. Procédé selon l'une quelconque des revendications 11 à 20, **caractérisé en ce que** ladite solution ou dispersion de matériau de chitosan est une solution aqueuse ou dispersion, comprenant de 0,1 à 40 %, de préférence de 1 à 10 % et plus préférablement environ 4 % en poids dudit matériau de chitosan.

22. Procédé selon l'une quelconque des revendications 11 à 21, **caractérisé en ce que** ladite solution ou dispersion de matériau de chitosan est appliquée en une quantité de 1 à 1000 mL, de préférence de 1 à 400 mL et plus préférablement de 1 à 150 mL de ladite solution ou dispersion de matériau de chitosan par mètre carré de ladite nappe de précurseur.

23. Procédé selon l'une quelconque des revendications 11 à 22, **caractérisé en ce que** ladite solution ou dispersion de matériau de chitosan est appliquée sur au moins une surface de ladite nappe de précurseur à travers au moins 40 %, de préférence 60 %, plus préférablement 80 % et le plus préférablement 100 % de la surface entière de ladite nappe de précurseur.

24. Article absorbant comprenant une feuille de dessus perméable aux liquides, une feuille de fond imperméable aux liquides et une âme absorbante, ladite âme absorbante comprenant un élément absorbant fabriqué selon l'une quelconque des revendications 11 à 23.

25. Article absorbant selon l'une quelconque des revendications 1 à 10 ou 24, **caractérisé en ce que** ladite feuille de fond imperméable aux liquides est une feuille de fond perméable à l'air permettant un transfert d'air et/ou de vapeur d'eau à travers.

26. Article absorbant selon l'une quelconque des revendications 1 à 10, 24 ou 25, **caractérisé en ce que** ledit article absorbant est un article absorbant pour l'hygiène féminine.
